# EUROPEAN PATENT APPLICATION

(11) **EP 2 233 094 A2**
(43) Date of publication of application: **29.09.2010**
(21) Application number: 10250608.6
(22) Date of filing: 26.03.2010
(51) Int. Cl.: A61B 17/34, A61B 19/00

(54) **Surgical access assembly including shield member**

(30) Priority: 27.03.2009 US 164010 P; 25.02.2010 US 712422
(71) Applicant: Tyco Healthcare Group LP, North Haven, CT 06473 (US)
(72) Inventor: Fischvogt, Gregory, CT 06514 (US)
(74) Representative: Soames, Candida Jane

(57) **Abstract**

A surgical access assembly includes a housing, an access member, a seal member, and a shield member. The access member extends distally from the housing, and includes a proximal end, a distal end, and a longitudinal opening for the reception of a surgical instrument. The seal member is positioned within the housing, and is adapted to removably receive the surgical instrument such that a substantially fluid-tight seal is formed therewith. The shield member is at least partially positioned externally of the housing, e.g. proximally thereof, and is configured, dimensioned, and adapted to inhibit contact between a practitioner and any fluid escaping from a surgical worksite. The shield member includes a rim and an opening extending through the shield member that is configured and dimensioned to receive the surgical instrument.

## Description

### CROSS REFERENCE TO RELATED APPLICATION

The present application claims the benefit of and priority to U.S. Provisional Application Serial No. 61/164,010 filed on March 27, 2009, the entire contents of which are incorporated herein by reference.

### BACKGROUND

### 1. Technical Field

The present disclosure relates to a surgical access assembly that is adapted for removable insertion into a patient's tissue. More specifically, the present disclosure relates to a surgical access assembly including a shield member that is configured, dimensioned, and adapted to inhibit contact between a practitioner and any fluid escaping from a surgical worksite during, e.g., laparoscopic or endoscopic procedures.

### 2. Background of the Related Art

In contemporary medical practice, many surgical procedures are performed through access asemblies, e.g., trocar and cannula assemblies. These devices incorporate narrow tubes or cannulae percutaneously inserted into a patient's body, through which one or more surgical instruments may be introduced to access a surgical worksite. Generally, such procedures are referred to as "endoscopic", and if performed in the patient's abdomen, in which case the procedure is referred to as "laparoscopic," or an arthroscopic procedure where surgery is performed on a joint ligament or the like - all procedures being characterized as minimally invasive.

In minimally invasive procedures, a fluid, such as an insufflation gas, is often communicated into the surgical worksite prior to the introduction of any surgical instrumentation to the patient's body. Introducing such fluids increases the pressure within the internal worksite, which thereby separates the patient's internal organs from the overlying tissue, e.g., the patient's skin, to increase visibility and/or create a larger, more accessible space in which to work. This increase in internal pressure also tends to result in the expulsion of fluids, including the insufflation gas, from the surgical worksite. In arthroscopic procedures, an irrigant such as a saline may be introduced to clean debris, bone chips etc, and/or provide greater access to the underlying bone or tissue area. The irrigant solution is also subject to emission toward the surgeon

Given the desirability of maintaining the integrity of the expanded surgical worksite, surgical access assemblies typically incorporate one or more seals through which the surgical instruments are inserted in an attempt to establish a substantially fluid-tight seal in both the presence and absence of surgical instrumentation, and curtail the escape of fluid. However, in the event that fluid does escape, either through or around the surgical access assembly, known surgical access assemblies are generally devoid of any mechanism that will deflect or redirect such fluid to inhibit contact with the practitioner.

### SUMMARY

In one aspect of the present disclosure, a surgical access assembly is disclosed that includes a housing, an access member, a seal member positioned within the housing that is adapted to removably receive a surgical instrument such that a substantially fluid-tight seal is formed therewith, and a shield member.

The access member extends distally from the housing, and includes a proximal end, a distal end, and a longitudinal opening for the reception of the surgical instrument

The shield member is at least partially positioned externally of the housing, e.g. proximally thereof, to deflect fluid communicated from the housing. The shield member includes a rim and an opening extending through the shield member that is configured and dimensioned to receive the surgical instrument.

The shield member may assume a variety of configurations without departing from the scope of the present disclosure, such as a substantially planar or arcuate configuration. In those embodiments where the shield member exhibits an arcuate configuration, the shield member may be curved in a distal direction such that an outer edge of the shield member is positioned distally of the opening extending through the shield member.

The shield member may be configured and dimensioned to frictionally engage an outer surface of the surgical instrument. Additionally, or alternatively, the shield member may include an o-ring positioned within the opening extending through the shield member that is at least partially formed from a substantially resilient material.

The shield member is repositionable between a first position, in which the opening defines a first transverse dimension that is smaller than an outer transverse dimension of the surgical instrument, and a second position, in which the opening defines a second transverse dimension that substantially approximates the outer transverse dimension of the surgical instrument.

In one embodiment, the shield member is at least partially formed of a substantially resilient material such that the shield member is normally biased towards the first position. In this embodiment, the shield member is repositioned from the first position to the second position upon insertion of the surgical instrument through the opening extending through the shield member.

In another embodiment, the shield member may be adapted for manual repositioning between the first position and the second position. For example, the shield member may include a collet mechanism that is at least partially positioned within the opening extending through the shield member, and movable between an expanded configuration, corresponding to the first position of the shield member, and a restricted configuration, corresponding to the second position of the shield member. In this embodiment, when the collet mechanism is in the expanded configuration, the surgical instrument is insertable through the collet mechanism, and when the collet mechanism is in the restricted configuration, the collect mechanism engages an outer surface of the surgical instrument to maintain the position of the surgical instrument relative to the shield member.

In another embodiment, the shield member may include a clamp mechanism that is at least partially positioned within the opening extending through the shield member, and movable between an open configuration, corresponding to the first position of the shield member, and a closed configuration, corresponding to the second position of the shield member. To facilitate movement of the clamp mechanism between the open configuration and the closed configuration, it is envisioned that the clamp mechanism may include a lever configured for manual manipulation.

In another aspect of the present disclosure, a surgical access assembly is disclosed that includes a housing, an access member extending distally from the housing with proximal and distal ends that is configured to removably receive a surgical instrument, and a shield member. The shield member is at least partially positionable proximally of the housing and about the surgical instrument to substantially interrupt the communication of fluid from the housing in a proximal direction.

In yet another aspect of the present disclosure, a method of assembling a surgical access assembly is disclosed that includes the steps of (i) providing a housing and an access member extending distally from the housing, wherein the access member includes a longitudinal opening for reception of a surgical instrument; (ii) providing a shield member including an opening extending therethrough that is configured and dimensioned to receive the surgical instrument; (iii) positioning the surgical instrument within the opening extending through the shield member; and (iv) positioning the surgical instrument within the elongate access assembly such that the shield member is at least partially positioned proximally of the housing to interrupt the communication of fluid from the housing in a proximal direction.

These and other features of the surgical access assembly and shield member disclosed herein will become more readily apparent to those skilled in the art through reference to the following detailed description of various embodiments of the present disclosure.

### BRIEF DESCRIPTION OF THE DRAWINGS

**FIG. 1** is a side, schematic view of a surgical access assembly including one embodiment of a shield member in accordance with the present disclosure;

**FIG. 2** is a cross-sectional view of the shield member seen in **FIG. 1****;**

**FIG. 3** is a top, perspective view of the shield member seen in **FIG. 1****;**

**FIG. 4** is a cross-sectional view of an alternate embodiment of the shield member seen in **FIG. 1** including a tapered opening extending therethrough;

**FIG. 5A** is a top, perspective view of an alternative embodiment of the shield member seen in **FIG. 1** including an elliptical opening extending therethrough;

**FIGS. 5B** and **5C** are top, perspective views of an alternative embodiments of the shield member seen in **FIG. 1** including linear openings extending therethrough;

**FIG. 6A** is a cross-sectional view of an alternate embodiment of the shield member seen in **FIG. 1** including a rim that is positioned proximally of the opening extending through the seal member;

**FIG. 6B** is a top, perspective view of an alternative embodiment of the shield member seen in **FIG. 1** including a substantially planar configuration;

**FIG. 7A** is a cross-sectional view of the shield member seen in **FIG. 1** shown in a first position prior to the insertion of a surgical instrument;

**FIG. 7B** is a cross-sectional view of the shield member seen in **FIG. 1** shown in a second position following insertion of a surgical instrument;

**FIG. 8** is a cross-sectional view of an alternative embodiment of the shield member seen in **FIG. 1** including an o-ring positioned within the opening extending through the shield member;

**FIG. 9** is a top, perspective view of the embodiment of the shield member shown in **FIG. 8****;**

**FIG. 10A** is a top, perspective view of the another embodiment of the shield member shown in **FIG. 1** including a collect mechanism at least partially positioned within the opening extending through the shield member, wherein the collet mechanism is illustrated in an expanded configuration with a surgical instrument inserted therethrough;

**FIG. 10B** is a top, perspective view of the embodiment of the shield member shown in **FIG. 10A** showing the collet mechanism in a restricted configuration with the surgical instrument inserted therethrough;

**FIG. 11** is a top, perspective view of the another embodiment of the shield member shown in **FIG. 1** including a clamp mechanism at least partially positioned within the opening extending through the shield member, wherein the clamp mechanism is illustrated with a surgical instrument inserted therethrough;

**FIG. 12A** is a top, perspective view of the embodiment of the shield member shown in **FIG. 11** illustrating the clamp mechanism in an open configuration; and

**FIG. 12B** is a top, perspective view of the embodiment of the shield member shown in **FIG. 11** illustrating the clamp mechanism in a closed configuration.

### DETAILED DESCRIPTION

In the drawings, and in the following description, wherein like references characters identify similar or identical elements, the term "proximal" should be understood as referring to the end of the disclosed surgical access assembly, or any component thereof, that is closest to the practitioner during proper use, while the term "distal" should be understood as referring to the end that is furthest from the practitioner. Additionally, the term "surgical instrument" should be understood to include any surgical instrument that may be employed during the course of surgical procedure, including but not limited to an obturator, a surgical stapling device, or the like, and the term "fluid" should be understood as referring to any fluid that may be present at a surgical worksite, such as insufflation gases, saline, (e.g., in an arthroscopic surgical procedure) or bodily fluids.

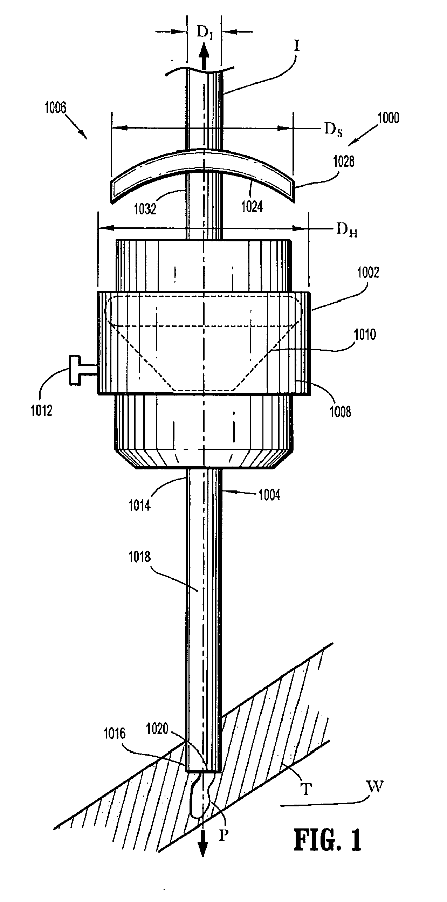

**FIG. 1** illustrates a surgical access assembly 1000 including a housing 1002, an elongate access member 1004, and a shield member 1006. The housing 1002 defines an internal cavity 1008 that is configured and dimensioned to accommodate a seal assembly 1010, and may be any structure suitable for this intended purpose. The seal assembly 1010 is adapted to removably receive a surgical instrument "I" such that a substantially fluid-tight seal is formed therewith. Illustrative examples of suitable seal assemblies are discussed in U.S. Patent No. 6,702,787 to Racenet the entire contents of which are incorporated by reference herein]. As depicted in **FIG. 1****,** the housing 1002 includes a port 1012 to permit the introduction of an insufflation gas into an internal surgical worksite "W" located beneath a patient's tissue "T."

The access member 1004 extends distally from the housing 1002 and is dimensioned for positioning within a percutaneous access point "P" formed in the patient's tissue "T." The access member 1004 includes respective proximal and distal ends 1014, 1016, and defines a longitudinal opening 1018. The longitudinal opening 1018 extends between the respective proximal and distal ends 1014, 1016 of the access member 1004 along a longitudinal axis "A," and is configured and dimensioned for the internal receipt of the surgical instrument "I." The access member 1004 defines an opening 1020 at the distal end 1016 thereof to allow the surgical instrument "I" to pass into the surgical worksite "W."

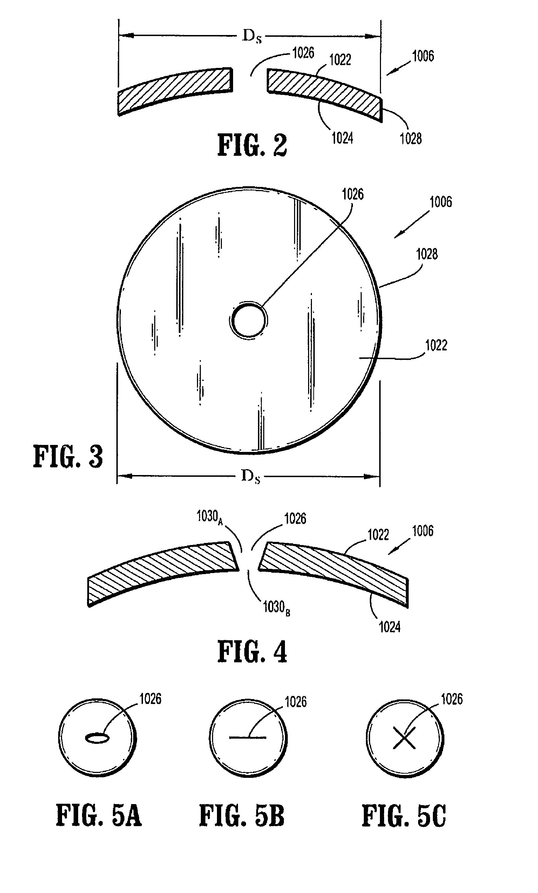

Referring now to **FIGS. 2** and **3** as well, the shield member 1006 will be discussed. The shield member 1006 defines an overall transverse dimension "D_{S}," and includes a proximal surface 1022, a distal surface 1024, an opening 1026, and an outer edge or rim 1028. Although the transverse dimension "D_{S}" of the shield member 1006 is illustrated as approximately equivalent to an outer dimension "D_{H}" of the housing 1002 **(****FIG. 1****)** of the surgical access assembly 1000, it shall be appreciated that the transverse dimension "D_{S}" of the shield member 1006 can be substantially larger or smaller dependent upon the particular circumstances of the surgical procedure in which the shield member 1006 is employed.

The opening 1026 extends between the respective proximal and distal surfaces 1022, 1024, and thus through the shield member 1006, to accommodate the insertion and removal of the surgical instrument "I" **(****FIG. 1****).** While the opening 1026 is illustrated as extending uniformly through the shield member 1006 in **FIGS. 1-3****,** alternate configurations for the opening 1026 are also contemplated. For example, as seen in **FIG. 4****,** the opening 1026 may be tapered to define a first aperture 1030_{A} on one surface of the shield member 1006, e.g., the proximal surface 1022, and a second aperture 1030_{B} on the other surface, e.g., the distal surface 1024, wherein the first aperture 1030_{B} and the second aperture 1030_{B} define differing transverse dimensions. Also, while the opening 1026 is depicted as substantially annular in **FIGS. 1-3****,** it is envisioned that the opening 1026 may define any suitable geometric configuration, e.g., elliptical **(****FIG. 5A****)** or linear **(****FIGS. 5B, 5C****).**

Referring again to **FIGS. 1-3****,** the rim 1028 of the shield member 1006 may be formed of the same material as the shield member 1006, or alternatively, may include a different material. It is further contemplated that the material comprising the rim 1028 may be substantially rigid or substantially non-rigid in nature.

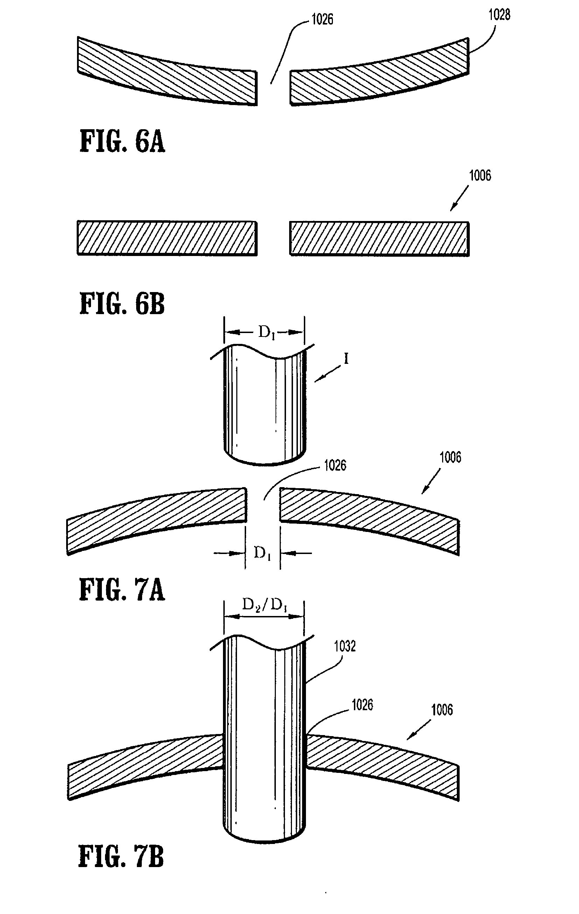

As shown in **FIG. 1****,** the shield member 1006 is separated from, and positioned externally of the housing 1002. However, in alternative embodiments of the surgical access assembly 1000, it is contemplated that a portion of the shield member 1006 may be attached to, or positioned within, the housing 1002. Additionally, although the shield member 1006 is illustrated as defining an arcuate configuration in which the rim 1028 of the shield member is positioned distally of the opening 1026 extending therethrough **(****FIGS. 1-3****),** other configurations are not beyond the scope of the present disclosure. For example, the rim 1028 of the shield member may be positioned proximally of the opening 1026 extending therethrough, as seen in **FIG. 6A****,** or the shield member 1006 may define a substantially planar configuration, as seen in **FIG. 6B****.**

The shield member 1006 may be formed from any suitable material, including but not being limited to elastomeric materials such as natural rubber, synthetic polyisoprene, butyl rubber, halogenated butyl rubbers, polybutadiene, styrene-butadiene rubber, nitrile rubber, hydrogenated nitrile rubbers, chloroprene rubber, ethylene propylene rubber, ethylene propylene diene rubber, epichlorohydrin rubber, polyacrylic rubber, silicone rubber, fluorsilicone rubber, fluoroelastomers, perfluoroelastomers, polyether block amides, chlorosulfonated polyethylene, ethylene-vinyl acetate, thermoplastic elastomers, thermoplastic vulcanizers, thermoplastic polyurethane, thermoplastic olefins, resilin, elastin, and polysulfide rubber. Forming the shield member 1006 from such a material permits the shield member 1006 to resiliently accommodate the insertion, manipulation, and removal of the surgical instruments "I" that may vary in size, e.g., in their outer dimension "D₁" **(****FIG. 1****).** Specifically, upon insertion of the surgical instrument "I", the shield member 1006 is moved from a first position **(****FIG. 7A****),** in which the opening 1026 extending through the shield member 1006 defines a first, or initial transverse dimension "D₁" that is smaller than the outer dimension "D₁" of the surgical instrument "I," to a second position **(****FIG. 7B****),** in which the opening 1026 defines a second, enlarged transverse dimension "D₂."

The resilient material comprising the shield member 1006 attributes a normal bias toward the first such that the shield member 1006 to return to the first position following removal of the surgical instrument "I" from the opening 1026. Additionally, the normal bias of the shield member 1006 allows the opening 1026 to realize a second transverse dimension "D₂" that substantially approximates the outer dimension "D₁" of the surgical instrument "I" **(****FIG. 7B****)** such that the shield member 1006 frictionally engages an outer surface 1032 of the surgical instrument "I" at the opening 1026 extending through the shield member 1006. This frictional engagement allows the shield member 1006 to be reliably positioned at any location along the portion of the surgical instrument "I" extending proximally of the housing 1002. Additionally, the frictional engagement between the shield member 1006 and the surgical instrument "I" allows the practitioner to reliably reposition the shield member 1006 if necessary. It is envisioned herein that the normal bias of the shield member 1006 towards the first position may be considerable enough to establish a substantially fluid-tight seal between the shield member 1006 and the outer surface 1032 of the surgical instrument "I".

Referring now to **FIGS. 1-3****,** **7A,** and **7B**, the use and function of the surgical access assembly 1000 **(****FIG. 1****)** during the course of a minimally invasive surgical procedure will be discussed. Initially, i.e., prior to beginning the procedure, the surgical instrument "I" is inserted through the opening 1026 in the shield member 1006 such that the shield member will be positioned proximally of the housing 1002 upon insertion of the surgical instrument "I" into the surgical access assembly 1000. Thereafter, the practitioner orients the surgical access assembly 1000 such that the distal end 1014 of the access member 1004 is positioned beneath the patient's tissue "T" within the surgical worksite "W" such as a joint or ligament in an arthroscopic procedure, or an insufflated body cavity. Fluid, such as an irrigant in the arthroscopic procedure or insufflation gas, can be introduced into the surgical worksite "W" through the port 1012 to increase visibility and/or create a larger, more accessible space in which to work or clear bone chips or debris in the joint area.

Following placement of the surgical access assembly 1000 as described above, fluid escaping from the surgical worksite "W" during the course of the surgical procedure, either around the surgical access assembly 1000 or therethrough, will be generally prevented from reaching the practitioner by the shield member 1006, thereby protecting the practitioner from potential exposure to objectionable substances. Following placement of the surgical access assembly 1000, the practitioner can insert the surgical instrument "I" into the surgical worksite "W" through the surgical access assembly 1000 to carry out the remainder of the procedure.

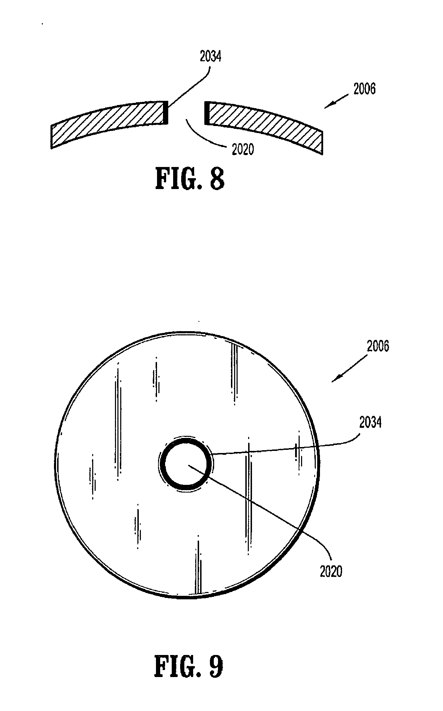

Referring now to **FIGS. 8-12B****,** alternative embodiments of the shield member 1006 **(****FIGS. 1-3****)** will be discussed. Each embodiment of the shield member shown in **FIGS. 8-12B** is substantially similar to the shield member 1006 discussed above with respect to **FIGS. 1-3****,** and accordingly, will only be discussed with respect to their differences therefrom.

With particular reference to **FIGS. 8** and **9****,** a shield member 2006 is disclosed that includes an o-ring 2034 positioned within the opening 2020 extending through the shield member 2006. Upon insertion of the surgical instrument "I" **(****FIG. 1****),** the o-ring 2034 facilitates engagement with the outer surface 1032 thereof such that a substantially fluid-tight seal is formed between the surgical instrument "I" and the shield member 2006. The o-ring 2034 can be formed from any material suitable for the intended purpose of establishing such a seal, including but not limited to rubber or polymeric materials.

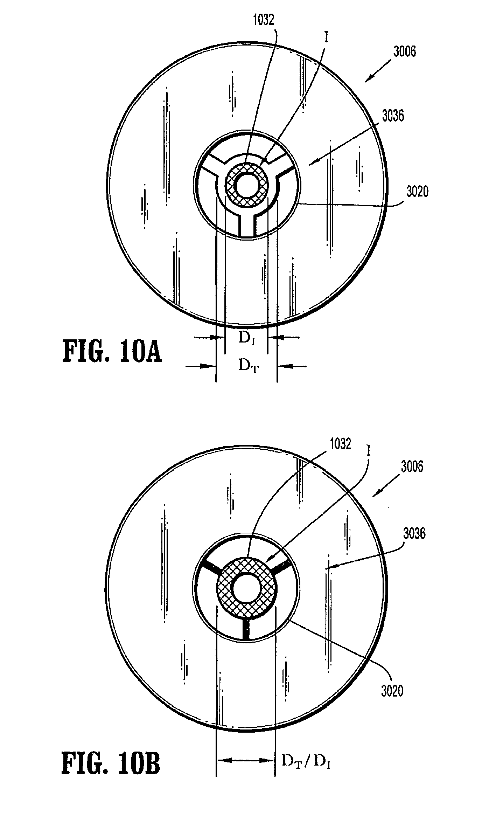

**FIGS. 10A** and **10B** illustrate a shield member 3006 that includes a collet mechanism 3036 positioned at least partially within the opening 3020. The collet mechanism 3036 allows for selective, manual repositioning of the shield member 3006 between the first position **(****FIG. 7A****)** and the second position **(****FIG. 7B****)**, and consequently, adjustment of the effective inner transverse dimension "D_{T}" of the opening 3020. Specifically, the collet mechanism 3036 is movable from an expanded configuration **(****FIG. 10A****)**, corresponding to the first position of the shield member 3006, and a restricted configuration **(****FIG. 10B****),** corresponding to the first position of the shield member 3006.

When the collet mechanism 3036 is in the expanded configuration, the effective inner transverse dimension "D_{T}" of the opening 3020 is large enough to allow for insertion of the surgical instrument "I" **(****FIG. 1****)** therethrough. However, when the collet mechanism is moved to the restricted configuration, the effective inner transverse dimension "D_{T}" of the opening 3020 is reduced to substantially approximate the outer dimension "D_{T}" of the surgical instrument "I" such that the collet mechanism 3036 engages the outer surface 1032 of the surgical instrument "I" to substantially maintain the position thereof relative to the shield member 3006.

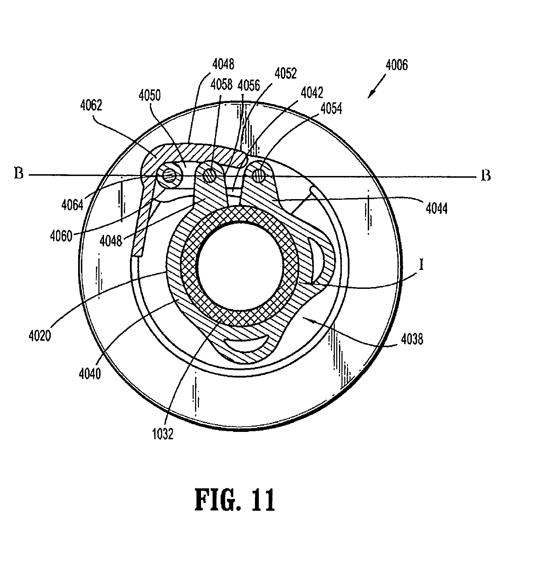

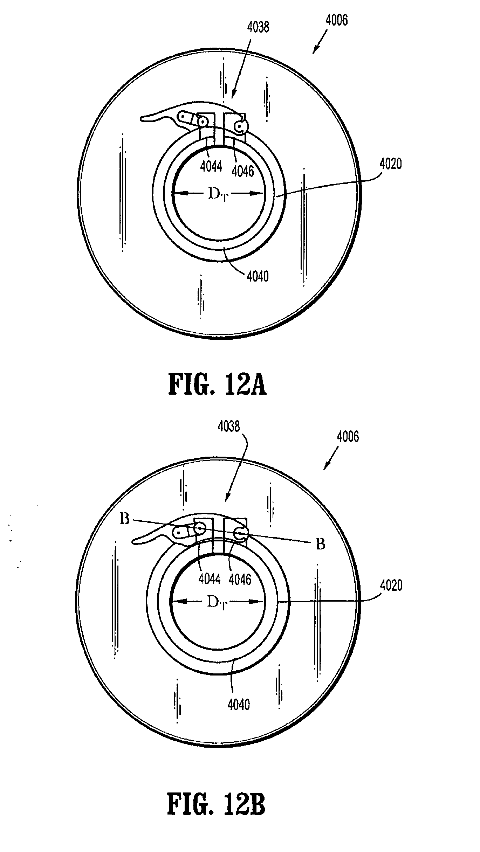

With reference now to **FIGS. 11-12B**, a shield member 4006 is illustrated that includes a clamp mechanism 4038 allowing for selective, manual repositioning of the shield member 4006 between the first position **(****FIG. 7A****)** and the second position **(****FIG. 7B****),** and consequently, adjustment of the effective inner transverse dimension "D_{T}" of the opening 4020. Specifically, the clamp mechanism 4038 is movable between an open configuration **(****FIG. 12A****),** corresponding to the first position of the shield member 4006, and a closed configuration **(****FIG.** 12B), corresponding to the first position of the shield member 4006.

The clamp mechanism 4038 includes a collar 4040 positioned at least partially within the opening 4020 extending through the shield member 4006. The collar 4040 defines a split 4042 **(****FIG. 11****)** which allows the collar 4040 to be flexible and compressible against the outer surface 1032 of the surgical instrument "I." First and second mounting projections, referred to respectively by reference characters 4044, 4046 are formed on either side of the split 4042. The clamp mechanism 4038 further includes a lever 4048 and a cam bar 4050. A first end 4052 of the lever 4048 is pivotally connected to the first mounting projection 4044 by a first pin 4054, and a first end 4056 of the cam bar 4050 is pivotally connected to the second mounting projection 4046 by a second pin 4058. A second end 4060 of the cam bar 4050 is also pivotally connected to a central portion 4062 of the lever 4048 by a third pin 4064.

The operation of the clamp mechanism will now be discussed with continuing reference to **FIGS. 11-12B**. Referring initially to **FIGS. 11** and **12A****,** when the lever 4048 is in a generally clockwise most position, the clamp mechanism 4038 is in the open configuration **(****FIG. 12A****)**, wherein the distance between the mounting projections 4044, 4046 is at a maximum, and the effective inner transverse dimension "D_{T}" of the opening 4020 **(****FIGS. 12A, 12B****),** which is defined by the collar 4040, is large enough to allow for insertion of the surgical instrument "I" **(****FIG. 11****)** through the opening 4020. As the lever 4048 is rotated counterclockwise, the second end 4060 of the cam bar 4050 moves through an arc, and drives the second mounting projection 4046 towards the first mounting projection 4044, thereby moving the clamping mechanism 4038 into the restricted configuration **(****FIG. 12B****).** In the restricted configuration, the effective inner transverse dimension "D_{T}" of the opening 4020 is reduced to substantially approximate the outer dimension "D_{I}" of the surgical instrument "I" such that the clamp mechanism 4038, specifically the collar 4040, engages the outer surface 1032 **(****FIG. 11****)** of the surgical instrument "I" to substantially maintain the position thereof relative to the shield member 4006.

When the third pin 4064 is in level alignment along line B-B with the first pin 4054 and the second pin 4058 **(****FIG. 11****),** the mounting projections 4044, 4046 are at their closest distance, and exert a maximum force against the outer surface 1032 of the surgical instrument "I." As the second end 4060 of the cam bar 4050 moves below line B-B, the distance between the mounting projections 4044, 4046 is increased, thereby increasing the force that must be applied to the lever 4048 to release the collar 4040 from the outer surface 1032 of the surgical instrument "I," and ensuring against inadvertent release of the clamp mechanism 4038.

The above description, disclosure, and figures should not be construed as limiting, but merely as exemplary of particular embodiments. It is to be understood, therefore, that the disclosure is not limited to the precise embodiments described, and that various other changes and modifications may be effected therein by one skilled in the art without departing from the scope or spirit of the disclosure. Additionally, persons skilled in the art will appreciate that the features illustrated or described in connection with one embodiment may be combined with those of another, and that such modifications and variations are also intended to be included within the scope of the present disclosure.

## Claims

1. A surgical access assembly comprising:
a housing;
an access member having proximal and distal ends and extending distally from the housing, the access member including a longitudinal opening for reception of a surgical instrument;
a seal member positioned within the housing, the seal member being adapted to removably receive the surgical instrument such that a substantially fluid-tight seal is formed therewith; and
a shield member including a rim and an opening extending through the shield member that is configured and dimensioned to receive the surgical instrument, at least a portion of the shield member being positioned externally of the housing to deflect fluid communicated from the housing.

2. The surgical access assembly of claim 1, wherein at least a portion of the shield member is positioned proximally of the housing.

3. The surgical access assembly of claim 1 or claim 2, wherein the shield member is substantially planar in configuration.

4. The surgical access assembly of claim 1 or claim 2, wherein the shield member exhibits an arcuate configuration.

5. The surgical access assembly of claim 4, wherein the shield member is curved in a distal direction such that the rim of the shield member is positioned distally of the opening.

6. The surgical access assembly of any preceding claim, wherein the shield member is configured and dimensioned to frictionally engage an outer surface of the surgical instrument.

7. The surgical access assembly of claim 6, wherein the shield member includes an o-ring positioned within the opening extending through the shield member, the o-ring being at least partially formed from a substantially resilient material.

8. The surgical access assembly of any preceding claim, wherein the shield member is repositionable between a first position, in which the opening defines a first transverse dimension smaller than an outer transverse dimension of the surgical instrument, and a second position, in which the opening defines a second transverse dimension that substantially approximates the outer transverse dimension of the surgical instrument.

9. The surgical access assembly of claim 8, wherein the shield member is adapted for manual repositioning between the first position and the second position.

10. The surgical access assembly of claim 9, wherein the shield member includes a collet mechanism positioned at least partially within the opening extending through the shield member, the collect mechanism being movable between an expanded configuration, corresponding to the first position of the shield member, in which the surgical instrument is insertable through the collet mechanism, and a restricted configuration, corresponding to the second position of the shield member, in which the collect mechanism maintains the position of the surgical instrument relative to the shield member through engagement with an outer surface of the surgical instrument.

11. The surgical access assembly of claim 9, wherein the shield member includes a clamp mechanism positioned at least partially within the opening extending through the shield member, the clamp mechanism being movable between an open configuration, corresponding to the first position of the shield member, and a closed configuration, corresponding to the second position of the shield member.

12. The surgical access assembly of claim 11, wherein the clamp mechanism includes a lever configured for manual manipulation to facilitate movement of the clamp mechanism between the open configuration and the closed configuration.

13. The surgical access assembly of any of claims 8 to 12, wherein the shield member is at least partially formed of a substantially resilient material such that the shield member is normally biased towards the first position, the shield member being repositioned from the first position to the second position upon insertion of the surgical instrument through the opening extending through the shield member.

14. A surgical access assembly comprising:
a housing;
an access member having proximal and distal ends and extending distally from the housing, the access member being configured to removably receive a surgical instrument; and
a shield member at least partially positionable proximally of the housing and about the surgical instrument to interrupt communication of fluid from the housing in a proximal direction.

15. A method of assembling a surgical access assembly including the steps of:
providing a housing and an access member extending distally from the housing, the access member including a longitudinal opening for reception of a surgical instrument;
providing a shield member including an opening extending therethrough configured and dimensioned to receive the surgical instrument;
positioning the surgical instrument within the opening extending through the shield member; and
positioning the surgical instrument within the elongate access assembly such that the shield member is at least partially positioned proximally of the housing to interrupt communication of fluid from the housing in a proximal direction.
